# EUROPÄISCHE PATENTANMELDUNG

(11) **EP 2 123 289 A1**
(43) Veröffentlichungstag der Anmeldung: **25.11.2009**
(21) Anmeldenummer: 09000738.6
(22) Anmeldetag: 21.01.2009
(51) Int. Cl.: A61K 35/16, G01N 1/40, C12M 1/24, C12M 1/26

(54) **Vorrichtung zur Herstellung einer biologisch aktiven Substanz**

(30) Priorität: 14.05.2008 DE 102008023545
(71) Anmelder: Roderfeld und Bora GmbH & Co. KG, 88662 Überlingen (DE)
(72) Erfinder: Roderfeld, Enno, Dr., 88630 Pfullendorf (DE)
(74) Vertreter: Daub, Thomas

(57) **Zusammenfassung**

Die Erfindung geht aus von einer Vorrichtung zur Herstellung zumindest einer biologisch aktiven Substanz mit zumindest einer Einheit (12), die einen Grundkörper (14) aufweist.

Es wird vorgeschlagen, dass die Einheit (12) ein Container (16) ist.

## Beschreibung

### Stand der Technik

Es ist bereits eine Vorrichtung zur Herstellung einer biologisch aktiven Substanz, die eine Einheit mit einem Grundkörper aufweist, bekannt.

Die Erfindung geht aus von einer Vorrichtung zur Herstellung einer biologisch aktiven Substanz nach dem Oberbegriff des Anspruchs 1.

### Vorteile der Erfindung

Die Erfindung geht aus von einer Vorrichtung zur Herstellung zumindest einer biologisch aktiven Substanz mit zumindest einer Einheit, die einen Grundkörper aufweist.

Es wird vorgeschlagen, dass die Einheit ein Container ist. Hierbei definiert ein Prozess einer "Herstellung" insbesondere eine Produktion, eine Gewinnung, eine Anreicherung, und/oder eine Vermehrung eines Stoffs, wie einer biologisch aktiven Substanz. Unter einer "biologisch aktiven Substanz" soll hier insbesondere ein Stoff und/oder ein Produkt verstanden werden, das einen Einfluss, wie beispielsweise eine Stimulation und/oder eine Hemmung einer Produktion und/oder eines Prozesses und/oder ein anderer, einem Fachmann als sinnvoll erscheinender Einfluss auf ein Element und/oder ein System, insbesondere auf ein biologisches System, hat. Hierbei stellt ein biologisches System insbesondere einen Organismus und/oder ein Lebewesen, wie einen Mikroorganismus (Bakterien, Pilze, Algen, Protozoen etc.), eine Pflanze, ein Tier dar, es sollen aber auch andere, dem Fachmann als sinnvoll erscheinende Elemente, wie etwa ein Virus, und/oder Systeme verstanden werden. Ein Einfluss bzw. ein Wirkmechanismus der Substanz beruht hierbei bevorzugt auf einer Wechselwirkung der Substanz mit zumindest einer Komponente des Organismus, wie z.B. Ionen, Spurenelemente, Nukleinsäuren, Aminosäuren, Peptide, Proteine (Hormone, Enzyme, Stoffwechselprodukte, Abbauprodukte etc.) und/oder zumindest einer sich im Organismus befindlichen körperfremden Substanz, wie z.B. Xenobiotika (Medikamente, Drogen, Toxine, Umweltgifte und/oder Lebensmittelzusatzstoffe etc.). In diesem Zusammenhang soll unter einer "Einheit" insbesondere ein Gebilde aus mehreren Komponenten, wie beispielsweise einem Grundkörper, verstanden werden, die eine Funktionseinheit bilden und/oder die nur unter Funktionsverlust der Einheit voneinander getrennt werden können. Ein "Grundkörper" bildet bevorzugt einen Hauptbestandteil der Einheit und definiert somit insbesondere eine Form und/oder eine Gestalt der Einheit. Diese Form ist bevorzugt röhrenförmig, in der Form eines Zylindermantels, an dem zumindest an einem Ende einer axialen Haupterstreckung des Zylindermantels eine Abschlussfläche, wie ein konisch verlaufendes Bodenteil, angeordnet ist. Es wären jedoch auch andere, dem Fachmann als zweckdienlich erscheinende Formen denkbar. Der Grundkörper besteht bevorzugt aus Metall, Glas und/oder besonders vorteilhaft aus einem Kunststoff, wie Polystyrol, Polypropylen, Polycarbonat, Polyethylen, Polyvinylchlorid, Polyethylenterephthalat und/oder aus einem anderen, dem Fachmann als zweckdienlich erscheinenden Material, insbesondere einem Material, das die Herstellung der biologisch aktiven Substanz unbeeinträchtigt lässt. Unter einem "Container" soll hier insbesondere ein Behältnis verstanden werden, das verschieden von einer Spritze ausgebildet ist und dessen Grundkörper insbesondere zumindest zum Großteil, im Vergleich insbesondere zu einem Beutel, starre Behältniswände bildet. Ferner sollte der Container insbesondere in zumindest einem Betriebszustand gegenüber einer Umgebung dicht bzw. undurchlässig für seinen Inhalt sein bzw. diesen vor Einflüssen aus der Umwelt und/oder der Umgebung schützen. Durch die Ausgestaltung der Einheit als Container, kann die Vorrichtung besonders stabil, sicher und bedienerfreundlich gestaltet werden. Eine gute, fehlerminimierende Handhabung, vor allem mit einer Bereitstellung der Möglichkeit eines unkomplizierten und sicheren Transports, wird gewährleistet.

Des Weiteren wird vorgeschlagen, dass der Container flügelfrei ausgestaltet ist. In diesem Zusammenhang soll unter "flügelfrei" insbesondere eine Ausgestaltung des Containers und insbesondere einer Fläche des Zylindermantels ohne einen, sich normalerweise in senkrechter Richtung zur Haupterstreckungsrichtung des Zylindermantels um mindestens eine Fingerbreite bzw. um mindestens 5 mm erstreckenden, angeformten und/oder an- oder aufgesteckten Flügel zur Fixierung einer Position eines Behältnisses verstanden werden. Dadurch kann ein kompakter Aufbau erreicht werden, der sich vorteilhaft zu einer kompakten Aufbewahrung und zu einem guten Transport beispielsweise mehrerer Container zusammen eignet.

Ferner ist es vorteilhaft, wenn der Container kolbenlos ausgestaltet ist. Hierbei soll unter "kolbenlos" insbesondere eine Ausgestaltung des Containers und insbesondere eines von dem Zylindermantel eingeschlossenen Hohlraums ohne einen, normalerweise in Richtung der Haupterstreckungsrichtung des Zylindermantels bzw. in dem von ihm eingeschlossenen Hohlraum beweglich angeordneten Kolben bzw. Stempel zur Erzeugung eines Drucks zum Einsaugen und/oder Auspressen von Gasen und/oder Flüssigkeiten in und/oder aus einem Hohlraum eines Behältnisses verstanden werden. Durch diese spezielle Ausgestaltung kann eine besonders stabile Vorrichtung bereitgestellt werden, die zudem günstig und konstruktiv einfach herstellbar ist.

In einer weiteren Ausgestaltung der Erfindung wird vorgeschlagen, dass zumindest eine Verschlussvorrichtung vorgesehen ist, die irreversibel mit dem Grundkörper verbunden ist. Hierbei soll unter einer "Verschlussvorrichtung" insbesondere eine Vorrichtung, wie eine Abdeckung in der Form eines Deckels oder einer Kappe, verstanden werden, die den Grundkörper in zumindest einem Betriebszustand, wie beispielsweise einer Transportsituation, gegen einen Ein- und/oder Austritt von einem Gas, wie beispielsweise Luft, einer Flüssigkeit und/oder einem Feststoff, verschließt und/oder abdichtet.

Ferner soll unter "irreversibel" verstanden werden, dass die Verschlussvorrichtung nur unter Funktionsverlust der Vorrichtung von dem Grundkörper getrennt werden kann. Hierbei sind sämtliche, dem Fachmann als sinnvoll erscheinende formschlüssige, kraftschlüssige und/oder stoffschlüssige Verbindungsarten, wie beispielsweise ein Rasten, ein Kleben, ein Schweißen, ein Sintern und/oder ein Löten zur Verbindung der Verschlussvorrichtung mit dem Grundkörper denkbar. Es wäre jedoch auch möglich, die Verschlussvorrichtung einstückig mit dem Grundkörper auszuführen. Die Verschlussvorrichtung besteht bevorzugt aus, Glas, Gummi, einem Kunststoff, wie Polystyrol, Polypropylen, Polycarbonat, Polyethylen, Polyvinylchlorid, Polyethylenterephthalat und/oder besonders vorteilhaft aus Metall und/oder aus einem anderen, dem Fachmann als zweckdienlich erscheinenden Material, insbesondere einem Material, das die Herstellung der biologisch aktiven Substanz unbeeinträchtigt lässt. Die Verschlussvorrichtung ist bevorzugterweise entlang der Haupterstreckungsrichtung des Zylindermantels an einem der Abschlussfläche des Grundkörpers abgewandten Ende angeordnet. Durch die Verschlussvorrichtung kann die Vorrichtung besonders bediensicher und bedienfreundlich gestaltet werden, zudem kann eine Bedingung, die in einem Hohlraum, den die Zylindermantelfläche einschließt, besteht, wie beispielsweise ein Unterdruck oder eine Sterilität, besonders einfach aufrechterhalten werden.

Ferner wird vorgeschlagen, dass die Vorrichtung zumindest eine Durchstechmembran aufweist. In diesem Zusammenhang soll unter einer "Durchstechmembran" insbesondere ein Teil der Einheit, des Grundkörpers und/oder der Verschlussvorrichtung verstanden werden, der aus einem Material gebildet ist, das in Bezug auf Festigkeit, Elastizität, Stoffdurchlässigkeit und/oder einer anderen, dem Fachmann als sinnvoll erscheinenden Eigenschaft eine andere Beschaffenheit aufweist, wie ein anderer Teil der Einheit, des Grundkörpers und/oder der Verschlussvorrichtung, wie beispielsweise ein Abdeckbereich der Verschlussvorrichtung. Dieses Material ist bevorzugt ein für Gase, Feststoffe und/oder für Flüssigkeiten zumindest weitgehend undurchlässiges Material, wie Gummi, Kunststoff und/oder Silikon, das leicht mit einem spitzen Gegenstand, wie einer Kanüle, die beispielsweise einen gängigen Außendurchmesser zwischen 1,5 und 0,5 mm (17 bis 25 Gauge) und eine Länge zwischen 20 und 100 mm aufweisen kann, durchstochen werden kann. Es wäre aber auch ein anderes, dem Fachmann als zweckdienlich erscheinendes Material, wie etwa Cellulose oder ein Derivat davon, denkbar. Die Durchstechmembran ist besonders bevorzugt in der Verschlussvorrichtung und insbesondere in einer Vertiefung der Verschlussvorrichtung angeordnet. Mit der Durchstechmembran ist konstruktiv einfach und bedienerfreundlich eine insbesondere sterile Entnahme von Material, wie Flüssigkeit und/oder Gas, aus einem Hohlraum des Containers möglich. Ferner kann eine Sterilität, zumindest der in der Verschlussvorrichtung angeordneten Durchstechmembran, vorteilhaft und konstruktiv einfach gewährleistet werden, wenn die Verschlussvorrichtung eine Abdeckungseinheit, wie beispielsweise eine abziehbare Folie bevorzugt aus Kunststoff, aufweist.

Des Weiteren kann es vorteilhaft sein, wenn die Vorrichtung zumindest ein Gasauslassventil und/oder ein Gaseinlassventil aufweist. In diesem Zusammenhang soll unter einem "Gaseinlassventil" bzw. einem "Gasauslassventil" insbesondere ein technisches Bauteil verstanden werden, das dazu dient, einen Aus- und/oder Einlass von Gasen, wie insbesondere von Luft, zu kontrollieren und/oder eine Fließrichtung zu steuern und/oder zu regeln. Das Gasauslassventil kann zu einer Vermeidung eines Überdrucks geöffnet werden bzw. öffenbar sein, um bei einer Befüllung eines Hohlraums des Containers mit einer Flüssigkeit die sich in dem Hohlraum befindlichen Gase entweichen zu lassen. Das Gaseinlassventil dient dahingegen einer besseren Entnahme der Flüssigkeit aus dem Container, um einen Unterdruck in dem Hohlraum bei der Flüssigkeitsentnahme zu verhindern. Das Gasauslassventil und/oder das Gaseinlassventil sind bevorzugt in der Verschlussvorrichtung angeordnet, es wäre jedoch grundsätzlich eine Anordnung im Grundkörper möglich. Das Gasauslassventil und/oder das Gaseinlassventil können insbesondere von einem Durchgangsventil, einem Absperrventil, einem Rückschlagventil, einem Rollmembranventil und/oder einem anderen, einem Fachmann als sinnvoll erscheinenden Ventil gebildet sein, das wiederum manuell, maschinell, elektromagnetisch, elektrisch, hydraulisch, pneumatisch und/oder mittels eines Mediums, wie Gas, Flüssigkeit und/oder einem anderen Medium und/oder auf eine andere Weise betätigbar sein kann. Vorteilhafterweise ist das Gasauslassventil und/oder das Gaseinlassventil als ein Rückschlagventil ausgebildet und insbesondere weist es eine Membran auf. Es wäre jedoch auch eine Ausgestaltung des Ventils mit Öffnungen, die durch eine manuelle Drehung eines Sperrrings freigegeben werden können, denkbar. Eine effiziente Platznutzung in der Verschlussvorrichtung kann erreicht werden, wenn die Durchstechmembran zwischen dem Gasauslassventil und dem Gaseinlassventil angeordnet ist. Hierbei soll unter der Wendung "zwischen dem Gasauslassventil und dem Gaseinlassventil" insbesondere eine räumliche Anordnung in Bezug auf das Gasauslassventil und das Gaseinlassventil verstanden werden. Durch eine Realisierung des Gasauslassventils und/oder des Gaseinlassventils kann konstruktiv einfach ein Druckausgleich für den Hohlraum des Containers bereitgestellt werden.

Weist der Container einen Hohlraum auf, in dem in zumindest einem Betriebszustand ein von einem Atmosphärendruck abweichender Druck herrscht, kann konstruktiv einfach eine gute Zufuhr des Blutes bzw. der Blutbestandteile mittels einer über eine Verbindungseinheit angeschlossene Blutentnahmevorrichtung in den Hohlraum erfolgen. Hierbei stellt der Betriebszustand bevorzugt einen Betriebszustand dar, bei dem der Hohlraum völlig unbefüllt von einer Flüssigkeit ist. Im Falle der Vorrichtung ist dies der Betriebszustand bevor die flüssigen Blutbestandteile in den Hohlraum einströmen. Ein Atmosphärendruck definiert hierbei einen mittleren Luftdruck der Atmosphäre, der beispielsweise auf Meereshöhe 101325 Pa beträgt. Ein abweichender Druck stellt hier einen Unterdruck und/oder ein Vakuum dar. In dieser Ausgestaltung kann bauteilsparend auf ein Gasauslassventil verzichtet werden.

Vorteilhafterweise ist an dem Grundkörper zumindest eine Verbindungseinheit angeordnet. Hierbei soll unter einer "Verbindungseinheit" insbesondere eine Einheit verstanden werden, die den Grundkörper des Containers mit einer weiteren Funktionseinheit, wie einer Blutentnahmevorrichtung, beispielsweise in der Form einer Spritze, eines Blutbeutels und/oder eines Infusionsschlauchs verbindet. Die Verbindungseinheit kann ein Verbindungsschlauch sein, wird jedoch bevorzugt von einem so genannten Luer-Konus und insbesondere bevorzugt von einem so genannten Luer-Lock gebildet. Die Verbindungseinheit ist bevorzugt an der Abschlussfläche des Grundkörpers angeordnet. Es wäre jedoch auch eine Anordnung an einer anderen Stelle denkbar. Bevorzugt ist die Verbindungseinheit einstückig mit dem Grundkörper ausgebildet, wobei einstückig insbesondere bedeutet, dass die Verschlussvorrichtung nur unter Funktionsverlust von dem Grundkörper getrennt werden kann. Grundsätzlich könnte eine Überwurfmutter des Luer-Locks aber auch als separates Bauteil ausgebildet sein, das auf den an dem Grundkörper angeformten Luer-Konus formschlüssig aufgesteckt werden kann. Durch die Anordnung der Verbindungseinheit kann konstruktiv einfach eine Zuführung und/oder Abführung von Stoffen in den Hohlraum des Containers erfolgen. Zudem kann die Verbindungseinheit eine Verschlusskappe aufweisen, die dazu vorgesehen ist, die Verbindungseinheit zu verschließen, wodurch ein Container bereitgestellt werden kann, der flexibel in verschiedenen Prozessschritten bedienerfreundlich nutzbar ist.

Zudem wird vorgeschlagen, dass die Vorrichtung zumindest einen Hohlraum aufweist, der zur sterilen Aufnahme von Blutbestandteilen vorgesehen ist. Unter einem "Hohlraum" soll hier insbesondere ein Raum verstanden werden, der zu einer Aufnahme eines Stoffs, wie eines Feststoffs, eines Gases und insbesondere einer Flüssigkeit und besonders vorteilhaft zur Aufnahme von Blut und/oder von Blutbestandteilen vorgesehen ist, und/oder der insbesondere dem Zweck dient, seinen Inhalt von seiner Umgebung bzw. seiner Umwelt zu isolieren. Hierbei stammen die Blutbestandteile aus Blut, hier insbesondere aus Vollblut. Die Blutbestandteile definieren hier zum einen einen Blutkuchen, demnach zelluläre Bestandteile des Bluts (rote und weiße Blutkörperchen), Blutplättchen und Gerinnungsfaktoren und zum anderen ein Blutserum, und zwar insbesondere eine geronnene Blutprobe ohne Blutkuchen. Das Blutserum enthält ca. 91 % Wasser und 7 % Proteine (Albumine und Globuline), die restlichen 2 % machen Elektrolyte, Nährstoffe und Hormone aus. In diesem Zusammenhang soll unter einer "sterilen Aufnahme" insbesondere die Einbringung und/oder Lagerung unter Bedingungen verstanden werden, bei denen die verwendeten Materialien, wie der Container, die Schläuche, die Kanülen oder andere, dem Fachmann als notwendig erachtete Gegenstände von Nukleinsäuren und/oder deren Fragmenten, Proteinen, Viren, lebenden Organismen, wie Mikroorganismen und/oder deren Sporen und/oder anderen verunreinigenden Substanzen befreit bzw. frei sind. Hierbei erfolgt die Sterilisation bevorzugt mittels Gammastrahlung. Durch den Hohlraum und die sterile Aufnahme kann eine Lagerung eines der Vorrichtung zugeführten Stoffs konstruktiv einfach erfolgen und eine Gefahr einer Kontamination bzw. Verunreinigung des Stoffs kann vorteilhaft vermieden werden.

Eine einfache Funktionsweise kann vorteilhaft erreicht werden, wenn die Vorrichtung zumindest einen Scherkörper aufweist. In diesem Zusammenhang soll unter einem "Scherkörper" insbesondere ein Körper und/oder eine Struktur verstanden werden, der und/oder die zu einer Aufbringung von Scherung bzw. von Scherkräften auf ein Probenobjekt, wie beispielsweise Blutzellen, vorgesehen ist. Hierbei definiert Scherung insbesondere eine Verformung des Probenobjekts unter Einwirkung einer Kraft, die tangential zu einer Seitenfläche des Probenobjekts wirkt. Aufgrund der Verformung tritt eine Stresssituation für das Probenobjekt bzw. die Blutzellen auf, woraufhin die Blutzellen eine biologisch aktive Substanz bilden und/oder ausschütten. Somit kann durch die Anordnung und Verwendung von Scherkörpern eine einfache, effektive und kostengünstige Stimulation einer Produktion von der biologisch aktiven Substanz, wie beispielsweise Stoffwechselprodukten, erreicht werden. Diese Stoffwechselprodukte bzw. der Container mit Scherkörpern sollen bevorzugt im Bereich der Behandlung von Entzündungen angewendet werden. Hierfür soll ein gewonnenes mit dem Stoffwechselprodukt angereichertes Blutserum insbesondere in ein entzündetes Gebiet, wie sämtliche am Körper befindlichen Gelenke, Bereiche in Wirbelsäulennähe, perineurale Bereiche, Muskeln, Sehnen, Bänder, Faszien und/oder andere, dem Fachmann als sinnvoll erscheinende Bereiche eingebracht bzw. eingespritzt werden. Eine Anwendung ist ungeeignet für eine Applikation direkt in die Blutbahn, also in Venen und Arterien und in ein Lymphsystem.

Bevorzugt sind die gebildeten Stoffwechselprodukte an Entzündungsreaktionen beteiligt. Dadurch kann der Container zur Herstellung und/oder Gewinnung von entzündungshemmenden Wirkstoffen genutzt werden. Ein bevorzugter entzündungshemmender Wirkstoff ist ein Interleukin-1-Rezeptorantagonist (IL-1Ra). Der Interleukin-1-Rezeptorantagonist ist ein Rezeptorantagonist, der spezifisch eine Wirkung eines Proteins, Interleukin-1 (IL1), neutralisiert. Interleukin-1 gehört als ein proentzündliches Schlüsselzytokin zu den wichtigsten entzündungsfördernden Signalstoffen des Immunsystems. Es wird nach einem Entzündungsreiz vor allem von Monozyten und Makrophagen gebildet. Entzündungsreize können Bakterien, Pilze, wie Hefen, Autoantigene, aber auch jede andere Art von Fremdpartikeln sein. Auch die Aufbringung der Scherkräfte durch die Scherkörper auf die Blutzellen stimuliert eine Entzündungsreaktion. Die proentzündlichen Effekte des Interleukin-1 werden durch Bindung an einem für Interleukin-1 spezifischen Interleukin-1-Rezeptor vermittelt. Der Interleukin-1-Rezeptorantagonist wird ebenfalls von Immunzellen freigesetzt und kommt im Körper als ein natürlicher Hemmstoff für das Interleukin-1 vor. Er spielt eine wesentliche Rolle bei einer Regulation eines durch Interleukin-1 vermittelten Entzündungsvorgangs. Eine Wirkung des Interleukin-1-Rezeptorantagonisten beruht darauf, dass er eine Wirkung des Interleukin-1 bremst und beendet, indem er anstelle von Interleukin-1 an den Interleukin-1-Rezeptor, der von einer Zielzelle exprimiert wird, an diese Zielzelle andockt und damit ein Anlagern des Interleukin-1 verhindert, wodurch das Interleukin-1 seine proentzündlichen Effekte nicht mehr vermitteln kann. Der Interleukin-1-Rezeptorantagonist ist also der antientzündliche Gegenspieler des Interleukin-1.

Eine bevorzugte Weiterbildung besteht darin, dass der zumindest eine Scherkörper in dem zumindest einen Hohlraum angeordnet ist, wodurch eine einfache Funktionsweise der Vorrichtung bzw. des Containers bereitgestellt werden kann, die gewährleistet, dass das Probenobjekt bzw. die Blutzellen konstruktiv einfach dem die Scherung hervorrufenden Scherkörper zugeführt werden können.

Es wird zudem vorgeschlagen, dass der zumindest eine Scherkörper von einer Perle gebildet ist. Hierbei soll unter einer "Perle" bevorzugt ein runder, kugelförmiger Körper verstanden werden. Durch eine Perlenform kann ein besonders effizienter und zudem schonend wirkender Scherkörper bereitgestellt werden. Grundsätzlich könnte der Scherkörper auch von einer anderen, dem Fachmann als sachdienlich erscheinenden Struktur gebildet sein. Solch eine Struktur könnte beispielsweise eine Profilierung, eine Aufrauhung und/oder eine andere Modifikation einer Oberfläche von und/oder in dem Grundkörper sein. Eine Vergrößerung dieser Oberfläche des Grundkörpers könnte auch durch ein in den Hohlraum des Grundkörpers eingebrachtes Labyrinthsystem zur Durchleitung von Blut und/oder Blutbestandteilen möglich sein. Die Scherung und/oder ein Stressen der Blutzellen könnten jedoch auch durch einen anderen Prozess, wie beispielsweise ein Schütteln des Containers und damit der Blutzellen, vermittelt werden.

Besonders vorteilhaft weist die Vorrichtung 240 Scherkörper in der Form von Perlen auf. Eine Anzahl von Perlen kann auch eine andere, dem Fachmann als sinnvoll erscheinende Menge haben. Der Scherkörper hat hierbei einen Durchmesser zwischen 2 und 5 mm, bevorzugt von 4 mm, wobei auch hier ein anderer, einem Fachmann als zweckdienlich erscheinender Durchmesser realisierbar wäre. Durch die Verwirklichung des Scherkörpers als Perle kann eine besonders raumsparende Anordnung insbesondere mehrerer Perlen in dem Hohlraum bereitgestellt werden.

Der Scherkörper bzw. die Perle kann aus einem Metall, einem Kunststoff und besonders vorteilhaft aus Glas sein, wodurch ein einfach herstellbarer, leichter und kostengünstiger Scherkörper erzielt werden kann, was direkt eine Bereitstellung einer kostengünstigen und bedienerfreundlichen Vorrichtung bzw. des Containers ermöglicht.

Zudem ist es vorteilhaft, wenn der Scherkörper eine glatte Oberfläche aufweist. Unter einer "glatten Oberfläche" soll hierbei insbesondere eine Oberfläche verstanden werden, die Erhebungen und/oder Vertiefungen mit einer von einer Haupterstreckung der Oberfläche abweichenden Erstreckung aufweist, wobei die Erhebungen und Vertiefungen kleiner sind als 0,1 mm. Durch diese Ausgestaltung kann eine schonende und effiziente Behandlung des Bluts bzw. der Blutbestandteile, wie der Blutzellen, bereitgestellt werden, durch die die Zellen einem moderaten Stress ausgesetzt werden können, der jedoch zu keiner Zerstörung der zellulären Strukturen, wie der Zellmembran, führt.

Des Weiteren wird vorgeschlagen, dass der zumindest eine Hohlraum ein Fassungsvolumen größer als 50 ml aufweist, vorteilhaft beträgt das Fassungsvolumen zwischen 50 und 100 ml und besonders bevorzugt zwischen 50 und 65 ml. Generell wäre jedoch auch ein anderes Fassungsvermögen zwischen 10 und 100 ml denkbar. Hierbei soll unter einem "Fassungsvolumen" insbesondere das Volumen verstanden werden, das maximal von einem Körper, wie einem Hohlraum, aufgenommen werden kann. Hierbei kann ein Füllmaterial fest, flüssig und/oder gasförmig sein. Wird einem Patienten ein Blutvolumen von 50 ml abgenommen, ergibt sich durch eine Behandlung des Bluts mit den Scherkörpern eine Konzentration an Interleukin-1-Rezeptorantagonist, die bis zu 140-fach höher ist als die Konzentration in einer unbehandelten Probe. Dadurch kann prozessual einfach eine effiziente Anreicherung eines Wirkstoffs in einem sinnvollen Volumen erreicht werden. Zusätzlich ist ein ausreichendes Volumen vorhanden, um gesundheitsfördernd eine Portionierung in kleinere Probenvolumina durchzuführen.

Diese portionierten Proben können aufbewahrt werden und können zu einem späteren Zeitpunkt Verwendung finden. Ferner muss dem Patienten nur einmal Blut abgenommen werden, was besonders bei einem Tier als Patienten bedienerfreundlich und schützend für einen Blutabnehmenden ist. Des Weiteren handelt es sich durch die Verwendung von Eigenblut des Patienten um eine schnelle Methode, um den Wirkstoff zu gewinnen.

In einer weiteren Ausgestaltung wird ein System zur Herstellung zumindest einer biologisch aktiven Substanz mit zumindest einer Vorrichtung vorgeschlagen, wobei zusätzlich zu einem Container zumindest ein weiteres Mittel zur Blutentnahme und/oder Blutaufbereitung als Bestandteil des Systems vorgesehen ist. Hierbei soll unter einem "Mittel zur Blutentnahme und/oder Blutaufbereitung" insbesondere ein Gegenstand, wie beispielsweise eine Spritze, eine Kanüle, ein Butterfly, eine Verschlusskappe, ein Verbindungsschlauch, eine Verbindungsvorrichtung, ein Sterilisierungsbedarfsgegenstand, ein Zentrifugationsträger, eine Bedienungsanleitung und/oder ein anderer, einem Fachmann als sinnvoll erscheinender Gegenstand verstanden werden, der in einem Prozess der Blutentnahme und/oder der Blutaufbereitung Verwendung finden kann. Hierbei definiert eine "Blutentnahme" insbesondere eine Gewinnung einer Blutprobe, bevorzugt aus einem Blutgefäß, wie beispielsweise einer Arterie, einer Vene und/oder einer Drosselvene eines Säugetiers. Die Gewinnung erfolgt bevorzugt mittels einer Kanüle, wobei hier unter einer "Kanüle" auch eine Butterflykanüle, eine Venenverweilkanüle und/oder ein anderes, dem Fachmann als zweckdienlich erscheinendes Blutabnahmemittel verstanden werden soll. Ein dafür geeignetes Säugetier stellt insbesondere ein Mensch, ein Affe, ein Hund, eine Katze, ein Unpaarhufer, wie ein Pferd, ein Paarhufer, wie ein Kamel, ein Rind und/oder ein Schwein, ein Rüsseltier, wie ein Elefant, dar. Es wäre jedoch auch für eine Blutentnahme an einem anderen tierischen Lebewesen denkbar. Unter einer "Blutaufbereitung" soll hier insbesondere ein Prozess verstanden werden, bei dem gewonnenes Blut und/oder Blutbestandteile einem Bearbeitungsprozess unterzogen werden. Dieser Bearbeitungsprozess umfasst bevorzugt eine Zuführung des Bluts in den Container bzw. den Hohlraum des Containers, eine Zentrifugation des Containers, gegebenenfalls mit einem speziell auf den Container abgestimmten Zentrifugationsträger und/oder einer Abnahme und/oder Lagerung von Blutbestandteilen aus dem Container. Es wären aber auch andere, dem Fachmann als zweckdienlich erscheinende Prozessschritte vorsehbar. Durch die Bereitstellung eines Systems mit einem weiteren Mittel kann besonders bedienerfreundlich ein Komplettpaket bereitgestellt werden, das zum Beispiel einem Anwender, wie einem Arzt, ein Zusammenstellen der benötigten Komponenten zur Durchführung des Prozesses abnimmt. Zusätzlich kann sichergestellt werden, dass alle notwendigen Komponenten während der Blutentnahme und/oder der Blutaufbereitung sofort zur Hand sind.

Ferner ist ein Zentrifugationsträger für einen Container vorgesehen, dessen Form zumindest auf eine äußere Form des Containers abgestimmt ist. In diesem Zusammenhang soll unter einem "Zentrifugationsträger" insbesondere ein Mittel, wie eine Zentrifugationsplatte oder insbesondere ein Zentrifugenröhrchen und/oder ein anderes, dem Fachmann als zweckdienlich erscheinendes Mittel verstanden werden, das für einen Zentrifugationsvorgang genutzt werden kann und das im Besonderen einen Gegenstand, wie beispielsweise den Container, in einer Zentrifuge während des Vorgangs der Zentrifugation lagert und/oder hält. Die "äußere Form" des Containers definiert hierbei insbesondere eine Außenkontur des Containers, welche im Wesentlichen durch eine Außenfläche des Zylindermantels mit der Abschlussfläche, einer Außenfläche des Luer-Locks und einer Außenfläche der Verschlussvorrichtung bestimmt wird. Durch die Vorsehung eines speziell auf den Container ausgelegten Zentrifugationsträgers kann eine besonders materialschonende Zentrifugation des Containers durchgeführt werden.

In einer alternativen Weiterbildung wird ein Gesamtsystem mit zumindest einem ersten System zur Herstellung zumindest einer biologisch aktiven Substanz und zumindest einem zweiten System zur Herstellung zumindest einer biologisch aktiven Substanz vorgeschlagen, wobei sich das erste System in zumindest einem Mittel von dem zweiten System unterscheidet. Hierbei definiert "erstes System" beispielsweise ein System zum Behandeln eines Menschen und ein "zweites System" definiert zum Beispiel ein System zum Behandeln eines großen Säugetiers, wie beispielsweise einem Pferd, einem Rind und/oder einem Kamel. Das System für den Menschen weist beispielsweise eine 50 ml Spritze und eine Kanüle und/oder einen Butterfly für die Blutentnahme, je einen Einmalsterilisationstupfer zur Desinfektion der Einstichstelle und der Durchstechmembran, einen Verbindungsschlauch zum Zuführen des Bluts in den Container, den Container mit einem Luer-Lock, eine Verschlusskappe zum Verschließen des Luer-Locks, einen Zentrifugationsträger für die Zentrifugation des Containers und mehrere, bevorzugt fünf, 10 ml Spritzen und zumindest eine Kanüle zu einem Aliquotieren des gewonnenen Blutserums auf. Im System für ein großes Säugetier, wie ein Pferd, kann hingegen zum Beispiel der Sterilisationstupfer fehlen, da das Immunsystem eines Pferdes weniger anfällig für Infektionen der Einstichstelle ist oder es könnte zwei 50 ml Spritzen, zwei Container und/oder eine angepasste Menge an Aliquotierspritzen (Bsp. 10-mal 10 ml, 5-mal 20 ml oder 2-mal 50 ml) beinhalten, da ein Pferd ein ca. fünf- bis zehnfach größeres Blutvolumen als der Mensch aufweist und demnach ein größeres Blutvolumen entnommen werden kann. Somit kann für jede zu behandelnde Säugerordnung und/oder -Gattung, wie beispielsweise Mensch, Affe, Hund, Katze, Paar- und Unpaarhufer, Rüsseltier, ein speziell abgestimmtes System vorgesehen sein, das gezielt auf die Bedürfnisse der Behandlung des Menschen und/oder des Tiers abgestimmt ist.

Es könnte auch ein Gesamtsystem vorgesehen sein, das verschiedene, sich in zumindest einem Mittel unterscheidende Systeme aufweist, die für unterschiedliche Vertriebsländer der Vorrichtung ausgestaltet sind und den dort jeweilig vorherrschenden Gegebenheiten, wie zum Beispiel der dort gesprochenen Sprache und/oder den dort herrschenden Temperaturen, angepasst sind. So ein Mittel könnte beispielsweise eine Bedienungsanleitung und/oder ein Kühlsystem sein.

Die Erfindung geht ferner von einem Verfahren zum Nutzen einer Vorrichtung aus, wobei erstens einem tierischen Organismus mit einer Blutentnahmevorrichtung Blutbestandteile entnommen werden, zweitens zumindest ein Teil dieser Blutbestandteile zumindest einem Container zugeführt wird und drittens zumindest ein Teil der Blutbestandteile in einem Hohlraum des Containers zumindest einer Stresssituation zur Bildung zumindest einer biologisch aktiven Substanz ausgesetzt werden kann. Unter einer "Stresssituation" soll hier insbesondere ein Ereignis verstanden werden, das sich von den normalen in vivo- Bedingungen der Blutbestandteile bzw. der Blutzellen unterscheidet. Dieses Ereignis kann eine Temperaturänderung, eine Druckänderung, eine Änderung eines chemischen Potentials, eine Änderung eines elektrischen Potentials, eine pH-Wertän-derung, eine Änderung einer Elektrolytkonzentration, eine mechanische Beanspruchung, wie eine Scherung und/oder ein anderes, dem Fachmann als sinnvoll erscheinendes Ereignis sein.

Für das Verfahren ist auch denkbar, dass weitere Schritte, wie beispielsweise eine Inkubation und/oder ein Bewegen und/oder ein Schütteln des Containers mit dem sich darin befindenden Blut, eine Zentrifugation des Containers zur Trennung der Blutbestandteile und des zumindest einen Scherkörpers voneinander, eine Gewinnung der biologisch aktiven Substanz, beispielsweise angereichert in einem Blutbestandteil, in der Form einer Abnahme von Aliquoten aus dem Hohlraume des Containers, bevorzugt über die Durchstechmembran, eine Lagerung der gewonnenen Aliquote und/oder andere, dem Fachmann als zweckdienlich erscheinende Schritte vorgesehen sind. Auch zusätzliche Zwischenschritte, wie beispielsweise eine Vorbehandlung des Bluts vor einer Zuführung in den Container, zwischen den aufgeführten Verfahrensschritten sind möglich. Durch das beschriebene Verfahren kann die Herstellung einer biologisch aktiven Substanz konstruktiv einfach, bediensicher und effizient erfolgen.

Die Vorrichtung ist dazu vorgesehen, nach den Richtlinien des Gesetzes über Medizinprodukte (Medizinproduktegesetz - MPG) zugelassen und eingesetzt zu werden.

### Zeichnung

Weitere Vorteile ergeben sich aus der folgenden Zeichnungsbeschreibung. In der Zeichnung ist ein Ausführungsbeispiel der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

### Es zeigen:

- Fig. 1: einen Container in einer Schnittdarstellung,
- Fig. 2: den Container aus Figur 1 von oben,
- Fig. 3: ein System mit einem Container der Figur 1 für eine Behandlung eines Menschen,
- Fig. 4: ein System mit einem Container der Figur 1 für eine Behandlung eines Pferds,
- Fig. 5: ein Gesamtsystem mit einem ersten und einem zweiten System nach der Figur 3 und 4,
- Fig. 6: einen Zentrifugationsträger für einen Container nach Figur 1,
- Fig. 7: den Zentrifugationsträger der Figur 6 mit einem darin aufgenommenen Container der Figur 1,
- Fig. 8: ein Flussdiagramm zum Verfahrensablauf,
- Fig. 9: einen Container nach Figur 1 im Prozess der Blutzufuhr und
- Fig. 10: einen Container nach Figur 1 im Prozess der Blutserumentnahme.

### Beschreibung des Ausführungsbeispiels

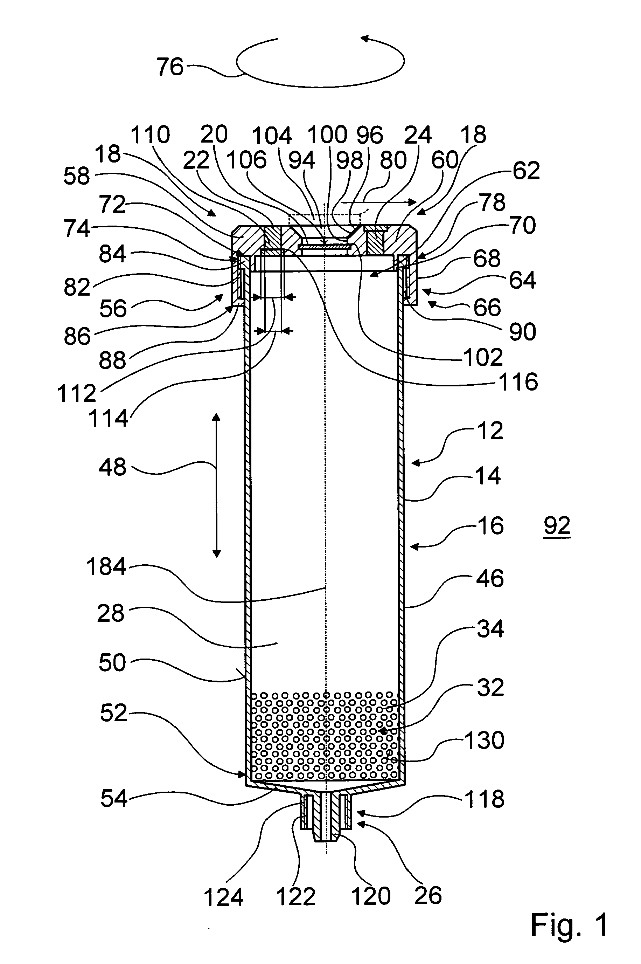

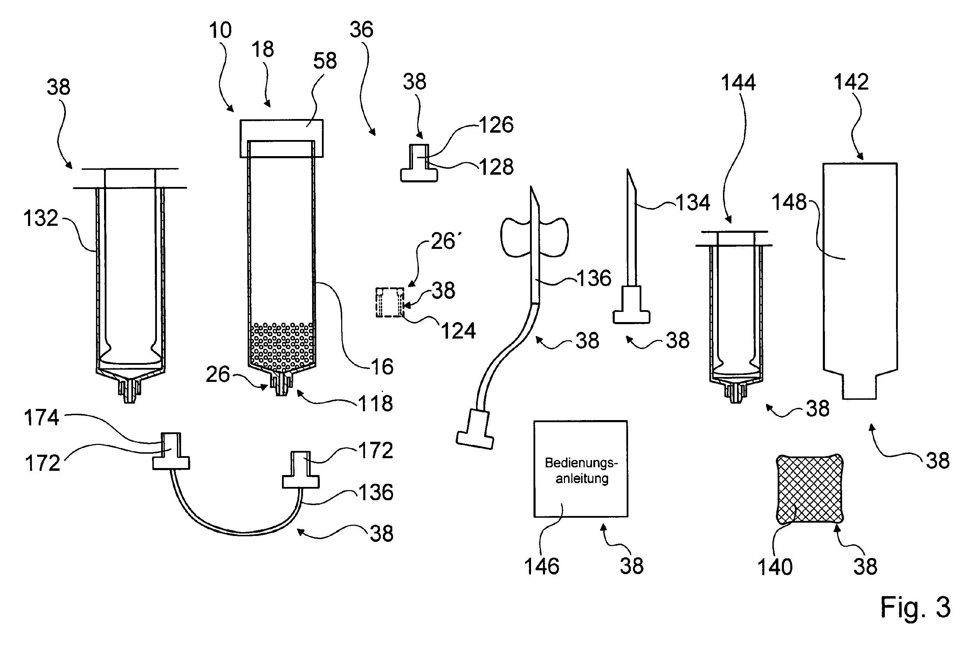

Figur 1 zeigt eine Vorrichtung 10 zur Herstellung zumindest einer biologisch aktiven Substanz. Die Vorrichtung 10 weist eine Einheit 12 auf, die einen Grundkörper 14 aus Kunststoff aufweist. Die Einheit 12 ist ein Container 16, der flügelfrei und kolbenlos ausgestaltet ist und demnach verschieden von einer Spritze, wie einer Blutentnahmespritze 132 (siehe Figur 3), ausgebildet ist. Der Grundkörper 14 weist eine starre Behältniswand in der Form eines Zylindermantels 46 auf, der sich entlang einer axialen Haupterstreckungsrichtung 48 des Containers 16 erstreckt und der mit einer Zylindermantelfläche 50 einen Hohlraum 28, der zur sterilen Aufnahme von Blutbestandteilen 30 vorgesehen ist, umschließt. An einem Ende 52 des Zylindermantels 46 des Grundkörpers 14 ist eine Abschlussfläche 54, die als ein konisch zu einer Mittelachse 184 des Grundkörpers 14 zulaufendes Bodenteil ausgebildet ist, angeordnet. Diese Abschlussfläche 54 ist einstückig mit dem Zylindermantel 46 ausgebildet.

Die Vorrichtung 10 bzw. der Container 16 weist ferner eine Verschlussvorrichtung 18 auf, die irreversibel mit dem Grundkörper 14 verbunden ist und die an einem weiteren Ende 56 des Zylindermantels 46 des Grundkörpers 14 angeordnet ist. Die Verschlussvorrichtung 18 bildet eine Abdeckung in der Form eines Deckels 58 aus Metall. Die Verschlussvorrichtung 18 bzw. der Deckel 58 weist einen Abdeckbereich 60 auf, der sich senkrecht zur Haupterstreckungsrichtung 48 des Containers 16 erstreckt und der eine Öffnung 62 des Hohlraums 28 vollständig abdeckt. Ferner weist die Verschlussvorrichtung 18 einen Verbindungsbereich 64 auf, der sich in einem Überdeckungsbereich 66 zwischen der Verschlussvorrichtung 18 und dem Grundkörper 14 parallel zur Haupterstreckungsrichtung 48 des Containers 16 und zum Zylindermantel 46 erstreckt.

Der Verbindungsbereich 64 ist gebildet von zwei sich in der Haupterstreckungsrichtung 48 des Containers 16 erstreckenden Ringsegmenten 68, 70, die einen Aufnahmebereich 72 für einen Endbereich 74 des Grundkörpers 14 bilden. Das Ringsegment 68 erstreckt sich in Umfangsrichtung 76 um eine äußere Oberfläche des Zylindermantels 46 und das Ringsegment 70 in Umfangsrichtung 76 in der inneren Oberfläche des Zylindermantels 46. An einem zum Abdeckbereich 60 weisenden bzw. an den Abdeckbereich 60 sich anschließendes Ende 78 des Ringsegments 68 ist eine sich in radialer Richtung 80 nach außen vom Zylindermantel 46 weg erstreckende Kontur 82 in der Form einer Rille eingebracht, in die ein Rastelement 84 in der Form eines Fortsatzes eingreift, das an dem Endbereich 74 des Zylindermantels 46 des Grundkörpers 14 angeformt ist und sich in radialer Richtung 80 nach außen zum Ringsegment 68 hin erstreckt. Die Kontur 82 stellt hierbei ein Innengewinde der Verschlussvorrichtung 18 dar, das mit dem Rastelement 84 in der Form eines Außengewindes des Grundkörpers 14 bei einem Aufschraubprozess des Deckels 58 auf den Grundkörper 14 bei einer Herstellung des Containers 16 in Eingriff kommt. In diesem hier nicht näher gezeigten Herstellungsprozess wird die Verschlussvorrichtung 18 zusätzlich zu dem Formschluss zwischen der Kontur 82 und dem Rastelement 84 durch einen weiteren Verfahrensschritt, wie beispielsweise einen thermischen Verbindungsschritt, unverlierbar und untrennbar an dem Grundkörper 14 fixiert.

An einem dem Ende 78 gegenüberliegenden bzw. dem Abdeckbereich 60 abgewandten Ende 86 des Ringsegments 68 ist ein sich in radialer Richtung 80 nach innen zum Zylindermantel 46 hin erstreckender Fortsatz 88 angeformt, der einen Zwischenraum 90, der im Überdeckungsbereich 66 zwischen dem Grundkörper 14 und dem Ringsegment 68 der Verschlussvorrichtung 18 gebildet wird, abschließt. Zudem dichtet der Fortsatz 88 den Zwischenraum 90 gegen einen Außenraum 92 bzw. gegenüber einer sich im Außenraum 92 befindlichen Luft ab. Zu einer Erhöhung der Luftdichtigkeit und einer Erhaltung einer Sterilität im Hohlraum 28 kann in den Zwischenraum 90 ein Füllstoff, wie ein Kleber, eingebracht sein. Ein Kleber dient zusätzlich der irreversiblen Verbindung der Verschlussvorrichtung 18 mit dem Grundkörper 14.

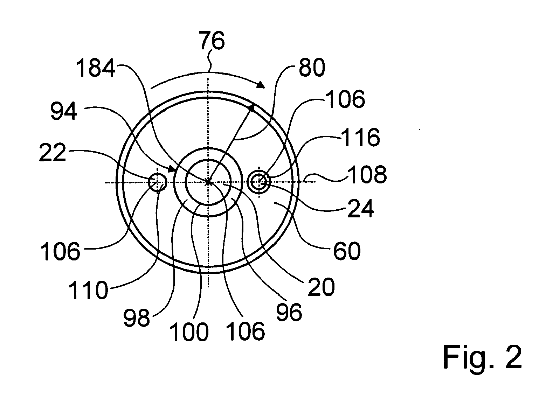

Zudem weist die Vorrichtung 10 bzw. der Container 16 eine Durchstechmembran 20 auf, die in dem Abdeckbereich 60 der Verschlussvorrichtung 18 mittig angeordnet ist (siehe Figur 2). Die Durchstechmembran 20 ist aus einer Gummischicht gebildet und im undurchstochenen Zustand undurchlässig für Feststoffe, Flüssigkeiten und/oder Gase. Hierfür ist in den Abdeckbereich 60 eine Öffnung 94 eingebracht, die eine Begrenzung 96 aufweist, die einen konisch verlaufenden Abschnitt 98 und einen zur Haupterstreckungsrichtung 48 parallel verlaufenden Abschnitt 100 bildet. In dem parallelen Abschnitt 100 ist eine in radialer Richtung 80 nach außen weisende Aussparung 102 eingebracht, die die Durchstechmembran 20 in ihrer Position senkrecht zur Haupterstreckungsrichtung 48 hält. Ferner kann die Verschlussvorrichtung 18 eine hier gestrichelt gezeigte Schutzeinheit 104 in der Form einer abziehbaren Folie mit Abziehlasche aus Kunststoff aufweisen, die dazu vorgesehen ist, die Durchstechmembran 20 vor ihrer Nutzung steril zu halten.

Die Vorrichtung weist zudem ein Gasauslassventil 22 und ein Gaseinlassventil 24 auf, die in der Verschlussvorrichtung 18 angeordnet sind und die als Rückschlagventile mit Membran ausgebildet sind. Das Gasauslass- und das Gaseinlassventil 22, 24 haben jeweils einen Mittelpunkt 106, die mit einem Mittelpunkt 106 der Öffnung 94, der auf der Mittelachse 184 des Containers liegt, auf einer Achse 108 liegen, wobei die Durchstechmembran 20 entlang der Achse 108 mittig zwischen dem Gasauslassventil 22 und dem Gaseinlassventil 24 angeordnet ist (siehe Figur 2). Das Gasauslass- und das Gaseinlassventil 22, 24 weisen einen sich entlang der Haupterstreckungsrichtung 48 des Containers 16 erstreckenden zylinderförmigen Abschnitt 110 und einen in seinem Durchmesser 112 gegenüber einem Durchmesser 114 des zylinderförmigen Abschnitts 110 breiteren scheibenförmigen Abschnitt 116 auf. In dem zylinderförmigen Abschnitt 110 ist die hier nicht näher dargestellte Membran zu einer Steuerung des Luftaus- bzw. Lufteintritts aus bzw. in den Hohlraum 28 angeordnet. Entlang der Haupterstreckungsrichtung 48 durchspannen das Gasauslass- und das Gaseinlassventil 22, 24 den Abdeckbereich 60 der Verschlussvorrichtung 18 in seiner gesamten axialen Länge. Das Gasauslass- und das Gaseinlassventil 22, 24 sind baugleich ausgestaltet und sind in entgegengesetzter Orientierung in die Verschlussvorrichtung 18 eingebracht. Sie stehen mit dem Hohlraum 28 in Kontakt bzw. es kann ein Gas- bzw. Luftaustausch durch das Gasauslass- und das Gaseinlassventil 22, 24 zwischen dem Hohlraum 28 und einem Außenraum 92 bzw. einer Umgebung des Containers 16 stattfinden.

Des Weiteren ist an dem Grundkörper 14 eine Verbindungseinheit 26 angeordnet. Die Verbindungseinheit 26 ist einstückig mit der Abschlussfläche 54, die an dem von der Verschlussvorrichtung 18 abgewandten Ende 52 des Zylindermantels 46 angeordnet ist, ausgeführt und in dieser in radialer Richtung 80 mittig angeordnet. Zudem wird die Verbindungseinheit 26 von einem Luer-Lock 118 gebildet, das sich aus einem Luer-Konus 120 und einer Wandung 122 bzw. von einer Überwurfmutter mit einem angeformten Gewinde in der Form eines Innengewindes 124 zusammensetzt. Auf dieses Innengewinde 124 kann beispielsweise zum Verschließen des Containers 16 eine Verschlusskappe 126 mit einem Außengewinde 128 aufgeschraubt werden (siehe Figur 3).

Der Hohlraum 28 weist ein Fassungsvolumen größer als 50 ml und in diesem Ausführungsbeispiel ein Fassungsvolumen von 60 ml auf. Zu einer Überwachung eines Befüllvorgangs der Vorrichtung 10 bzw. des Containers 16 kann an der Zylindermantelfläche 50 eine hier nicht näher gezeigte Skalierung, beispielsweise in 10 ml-Schritten, vorgesehen sein.

Zudem weist die Vorrichtung 10 eine Vielzahl, insbesondere in diesem Ausführungsbeispiel eine Anzahl von 240 Scherkörpern 32 auf, die in dem Hohlraum 28 des Containers 16 angeordnet sind. Von den Scherkörpern 32 sind zur Wahrung der Übersichtlichkeit nur einzelne in der Figur 1 gezeigt. Die Scherkörper 32 sind von Perlen 34 mit einem Durchmesser von 4 mm gebildet, die aus Glas gefertigt sind und eine glatte Oberfläche 130 aufweisen. Die Scherkörper 32 nehmen etwa ein Volumen von 10 ml des 60 ml-Volumens des Containers 16 ein.

In der Figur 3 ist ein System 36 zur Herstellung einer biologisch aktiven Substanz mit einer Vorrichtung 10 in der Form eines Containers 16 gezeigt, wobei die Bestandteile des Systems 36 nicht maßstabsgetreu wiedergegeben sind. Als weitere Bestandteile des Systems 36 zusätzlich zu dem Container 16 sind weitere Mittel 38, wie eine Blutentnahmespritze 132, eine Kanüle 134, ein Butterfly 136, ein Verbindungsschlauch 138, zwei Einmalsterilisationstupfer 140, eine Verschlusskappe 126, ein Zentrifugationsträger 142, fünf Aliquotierspritzen 144 und eine Bedienungsanleitung 146, zur Blutentnahme und/oder Blutaufbereitung vorgesehen. Jedes Mittel 38 ist nur 1-mal gezeigt. Alternativ zu der einstückigen Ausführung des Luer-Locks 118 mit dem Container 16 könnte auch eine separate, hier gestrichelt gezeigte Verbindungseinheit 26', ausgestaltet als aufsteckbare Überwurfmutter mit Innengewinde 124, vorgesehen sein. Das gezeigte System 36 ist für eine Behandlung eines Menschen ausgelegt.

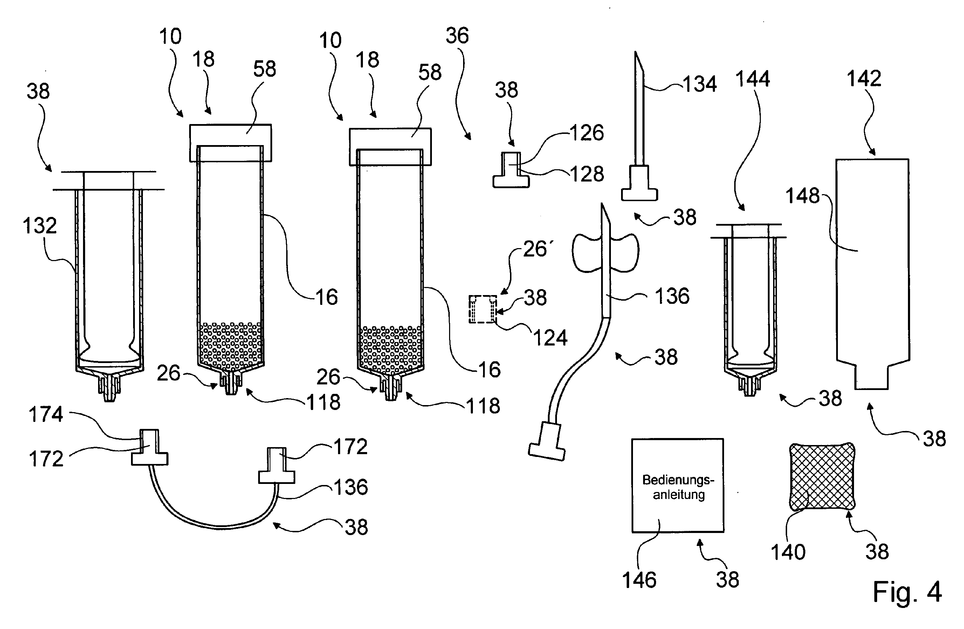

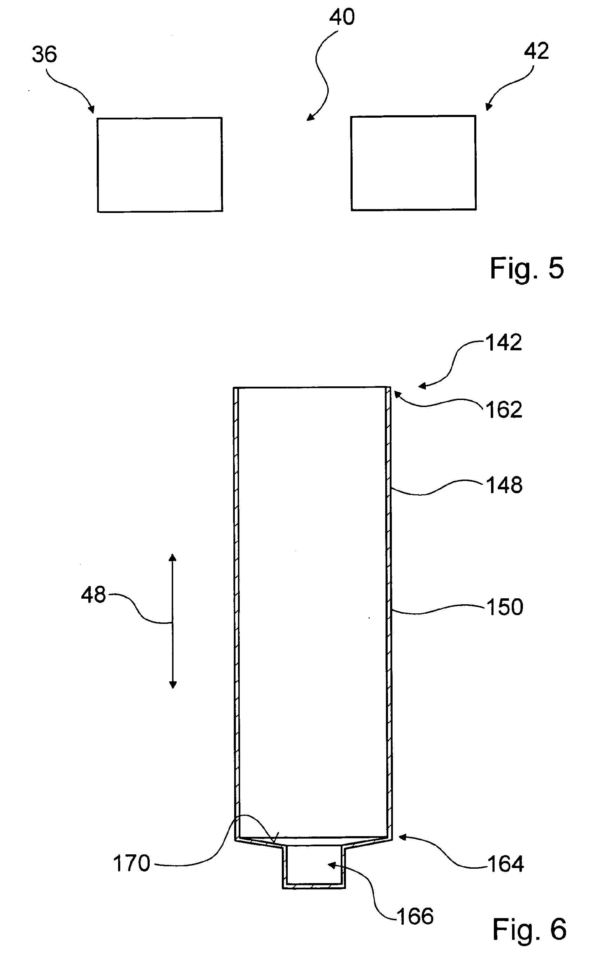

Die Figur 4 hingegen zeigt ein zweites System 42 zur Behandlung eines Pferds. Dieses zweite System 42 unterscheidet sich von dem ersten System 36 in mehreren Mitteln 38. Die beiden Systeme 36, 42 bilden somit ein Gesamtsystem 40 (siehe Figur 5). Das zweite System 42 weist zwei Container 16, zwei Blutentnahmespritzen 132 (je nur eine gezeigt) und zehn Aliquotierspritzen 144 (je nur eine gezeigt) auf, es ist somit dafür ausgelegt, die doppelte Menge an Blut gegenüber dem System 36 für den Menschen aufzuarbeiten.

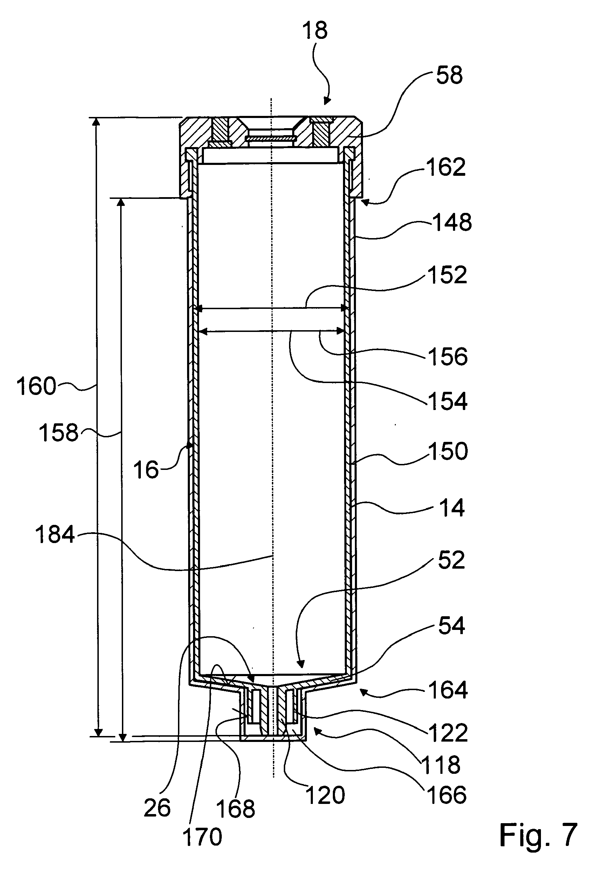

Ein speziell für den Container 16 ausgelegter Zentrifugationsträger 142 in der Form eines Zentrifugationsröhrchens 148 ist in der Figur 6 gezeigt. Die Form des Zentrifugationsträgers 142 ist auf eine äußere Form des Containers 16 abgestimmt. Hierbei weist des Zentrifugationsröhrchen 148 einen Grundkörper 150 auf, der sich, wie der Grundkörper 14 des Containers 16, in der Haupterstreckungsrichtung 48 des Containers 16 erstreckt. Ein Durchmesser 152 des Grundkörpers 150 ist breiter als ein Durchmesser 154 des Grundkörpers 14, so dass der Container 16 in seiner Breite 156 völlig von dem Zentrifugationsträger 142 aufgenommen wird. Eine axiale Länge 158 des Grundkörpers 150 ist kürzer als eine axiale Länge 160 des Grundkörpers 14, so dass im eingeführten Zustand des Containers 16 in den Zentrifugationsträger 142 die Verschlussvorrichtung 18 bzw. der Deckel 58 ein Ende 162 des Zentrifugationsträgers 142 überragt (siehe Figur 7). An einem dem Ende 162 gegenüberliegenden Ende 164 des Zentrifugationsröhrchens 148 ist eine Aufnahme 166 für das Ende 52 bzw. die Abschlussfläche 54 bzw. die Verbindungseinheit 26 bzw. das Luer-Lock 118 und die Verschlusskappe 126 angeformt. Diese Aufnahme 166 ist in ihrer Form so gestaltet, dass eine Außenkontur 168, gebildet durch die Elemente 26, 54, 118, 126 am Ende 52 des Containers 16 stabilisierend an einer Innenkontur 170 der Aufnahme 166 anliegt.

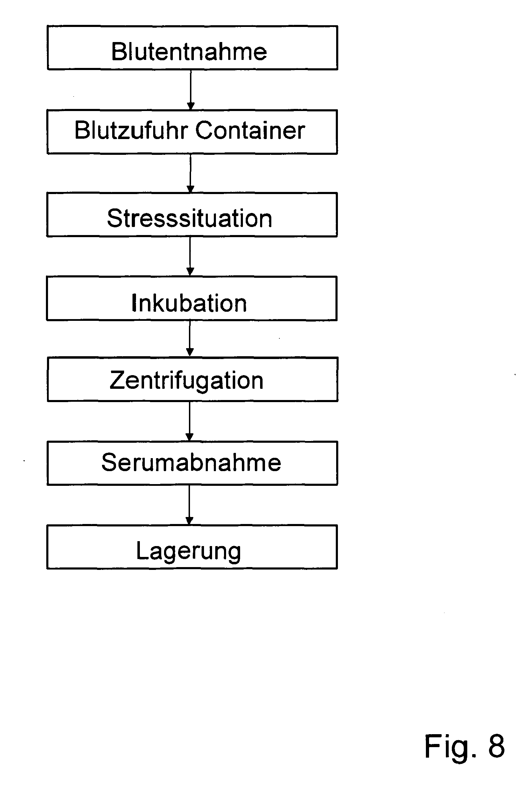

Im Folgenden wird ein Verfahren zum Nutzen einer Vorrichtung 10 und eines Systems 42 beschrieben, wobei nur eine Blutentnahme und eine Aufarbeitungsprozedur beschrieben ist, obwohl das System 42 Materialien für mehrere Durchführungen bereit stellt. Das Verfahren ist in der Figur 8 als Flussdiagramm dargestellt.

Vor einem Einsatz der Vorrichtung 10 bzw. des Containers 16 werden einem tierischen Organismus bzw. einem Patienten, wie einem Pferd, mit einer Blutentnahmevorrichtung 44 Blut bzw. Blutbestandteile 30 entnommen. Hierbei wird, nach einem dem Fachmann bekannten Vorgang, dem Patienten mit einer Blutentnahmespritze 132 und einer darauf aufgesteckten Kanüle 134 oder einem Butterfly 136 an einer vorher mit einem Einmalsterilisationstupfer 140 desinfizierten Einstichstelle ein Volumen von 50 ml Blut entnommen (nicht gezeigt).

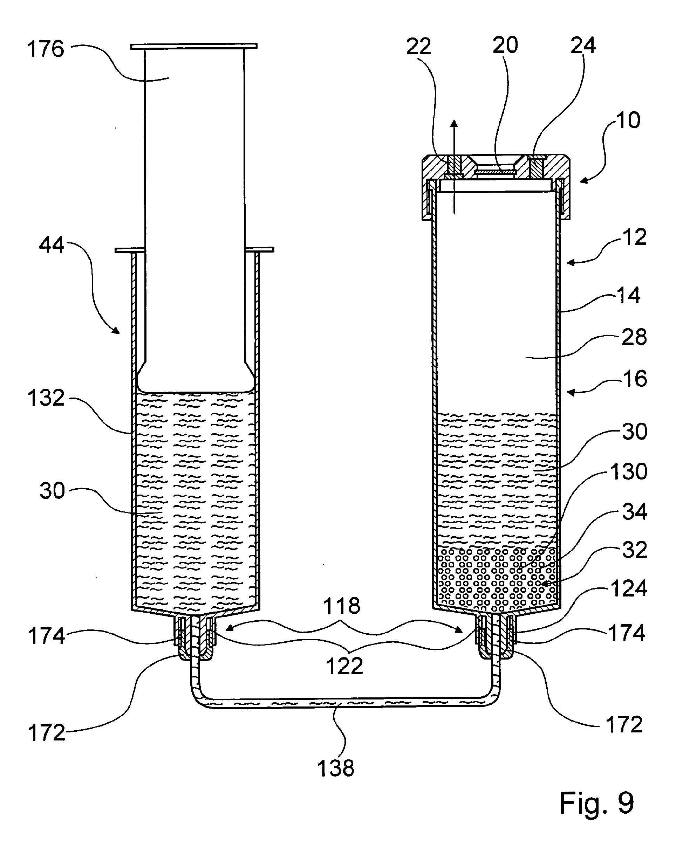

Anschließend werden diese Blutbestandteile 30 dem Container 16 zugeführt, wofür ein Verbindungsschlauch 138 mit zwei Koppelstellen 172 verwendet wird. Vor der Zufuhr befinden sich in dem Hohlraum 28 des Containers 16 nur die Scherkörper 32 und Luft (nicht gezeigt). Der Verbindungsschlauch 138 wird mit einem Außengewinde 174 je einer der Koppelstellen 172 auf das Innengewinde 124 des Luer-Locks 118 der Blutentnahmespritze 132 bzw. des Luer-Locks 118 des Containers 16 aufgeschraubt. Im Anschluss werden die Blutbestandteile 30 durch ein Aufbringen eines Drucks auf einen Kolben 176 der Blutentnahmespritze 132 durch den Verbindungsschlauch 138 in den Container 16 gedrückt, wobei die sich im Hohlraum 28 befindliche Luft über das Gasauslassventil 22 entweichen kann (siehe Figur 9). Bei dieser Zuführung wird ein Teil der Blutbestandteile 30 in dem Hohlraum 28 des Containers 16 einer Stresssituation zur Bildung einer biologisch aktiven Substanz ausgesetzt, indem die sich im Blut befindenden Blutzellen bei der Blutzufuhr durch den Einfülldruck mit erhöhtem Druck an den Scherkörpern 32 vorbeigedrängt werden. Diese Interaktion der Blutzellen mit den Perlen 34 bzw. deren glatter Oberfläche 130 bedeutet für die Blutzellen eine Stresssituation, die einem Entzündungsprozess nahe kommt. Als Reaktion auf diese Stimulation bilden die Blutzellen Stoffwechselprodukte (Interleukin-1 und Interleukin-1-Rezeptoranta-gonist), die bei Entzündungsreaktionen in Erscheinung treten.

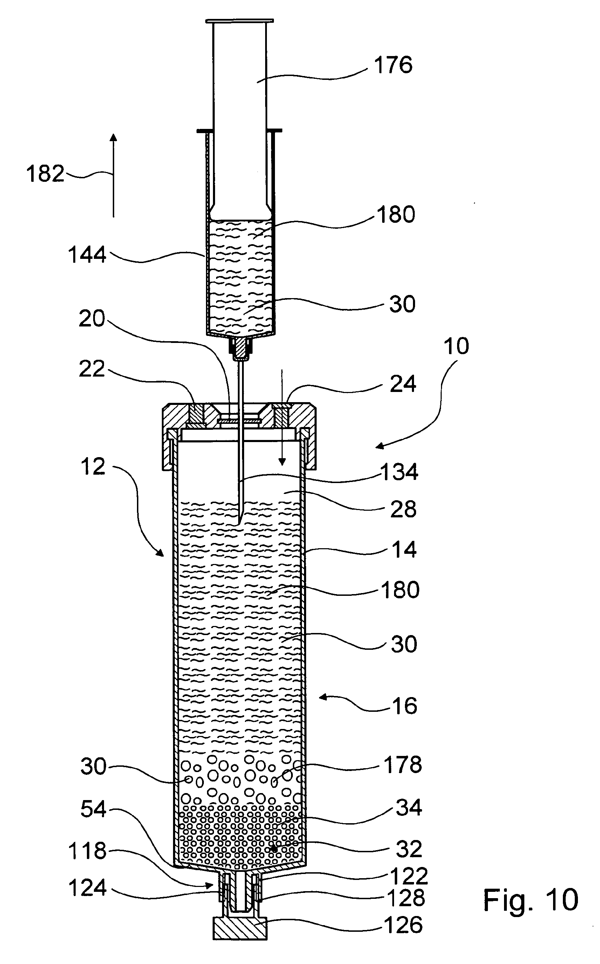

Nach einer Abnahme des Verbindungsschlauchs 138 wird eine Verschlusskappe 126 mit ihrem Außengewinde 128 auf das Innengewinde 124 des Luer-Locks 118 aufgeschraubt, um den Container 16 zu verschließen (siehe Figur 10).

Um eine ausreichende Anreicherung des Bluts mit diesen Stoffwechselprodukten bzw. bevorzugt die Anreicherung des entzündungshemmenden Interleukin-1-Rezeptorantagonisten zu erreichen, wird das abgenommene Blut in der Vorrichtung 10 für 2 bis 8 Stunden bei 37°C in einem Brutschrank inkubiert. Grundsätzlich wäre es jedoch auch möglich, auf die Inkubation zu verzichten und sie gegebenenfalls durch ein äquivalentes Mittel, wie beispielsweise ein Schütteln des Containers 16, zu ersetzen.

Nach der Inkubationszeit wird die Vorrichtung 10 aus dem Brutschrank entnommen und mit einer Zentrifuge in dem Zentrifugationsträger 142 bei Raumtemperatur für eine Zeitspanne zentrifugiert, die dazu ausreicht, die Scherkörper 32 bzw. feste Blutbestandteile 30, einen Blutkuchen 178 zu sedimentieren (beispielsweise 10 Minuten bei 5000 rpm). Nach der Zentrifugation haben sich drei Schichten gebildet. An der Abschlussfläche 54 des Containers 16 haben sich erst die schweren Scherkörper 32 abgesetzt, danach folgen die zellulären Blutbestandteile 30 bzw. der Blutkuchen 178 und als letztes haben sich flüssige Blutbestandteile 30, ein Blutserum 180, angesammelt (siehe Figur 10).

Nachfolgend wird, wie in Figur 10 ebenso gezeigt ist, das Blutserum 180 über eine Durchstechmembran 20 entnommen. Hierfür wird die Durchstechmembran 20 mit einem Einmalsterilisationstupfer 140 desinfiziert (nicht gezeigt) und es wird eine Kanüle 134 einer Aliquotierspritze 144 durch die Durchstechmembran 20 in den mit Blutserum 180 befüllten Hohlraum 28 des Containers 16 eingeführt bis die Kanüle 134 in das Blutserum 180 eintaucht. Durch ein Zurückziehen eines Kolbens 176 der Aliquotierspritze 144 in axialer Richtung 182 werden nun die flüssigen Blutbestandteile 30 bzw. das Blutserum 180 aus dem Hohlraum 28 des Containers 16 entnommen. Zur Erleichterung der Serumabnahme wird der Hohlraum 28 zum Druckausgleich über das Gaseinlassventil 24 mit Luft befüllt. Der Abnahmeschritt wird so lange wiederholt, bis das gesamte Blutserum 180 aus dem Hohlraum 28 entnommen wurde. Die so gewonnenen Aliquotierspritzen 144 mit dem mit Interleukin-1-Rezeptorantagonist angereicherten Blutserum 180 eines Patienten können nun zur Rückführung des Blutserums 180 in den Patienten genutzt werden. Dazu können sie bis zu 7 Monate bei -18 bis -20°C gelagert werden.

### Bezugszeichen

- 10: Vorrichtung
- 12: Einheit
- 14: Grundkörper
- 16: Container
- 18: Verschlussvorrichtung
- 20: Durchstechmembran
- 22: Gasauslassventil
- 24: Gaseinlassventil
- 26: Verbindungseinheit
- 28: Hohlraum
- 30: Blutbestandteil
- 32: Scherkörper
- 34: Perle
- 36: System
- 38: Mittel
- 40: Gesamtsystem
- 42: System
- 44: Blutentnahmevorrichtung
- 46: Zylindermantel
- 48: Haupterstreckungsrichtung
- 50: Zylindermantelfläche
- 52: Ende
- 54: Abschlussfläche
- 56: Ende
- 58: Deckel
- 60: Abdeckbereich
- 62: Öffnung
- 64: Verbindungsbereich
- 66: Überdeckungsbereich
- 68: Ringsegment
- 70: Ringsegment
- 72: Aufnahmebereich
- 74: Endbereich
- 76: Umfangsrichtung
- 78: Ende
- 80: radiale Richtung
- 82: Kontur
- 84: Rastelement
- 86: Ende
- 88: Fortsatz
- 90: Zwischenraum
- 92: Außenraum
- 94: Öffnung
- 96: Begrenzung
- 98: Abschnitt
- 100: Abschnitt
- 102: Aussparung
- 104: Schutzeinheit
- 106: Mittelpunkt
- 108: Achse
- 110: Abschnitt
- 112: Durchmesser
- 114: Durchmesser
- 116: Abschnitt
- 118: Luer-Lock
- 120: Luer-Konus
- 122: Wandung
- 124: Innengewinde
- 126: Verschlusskappe
- 128: Außengewinde
- 130: Oberfläche
- 132: Blutentnahmespritze
- 134: Kanüle
- 136: Butterfly
- 138: Verbindungsschlauch
- 140: Einmalsterilisationstupfer
- 142: Zentrifugationsträger
- 144: Aliquotierspritze
- 146: Bedienungsanleitung
- 148: Zentrifugationsröhrchen
- 150: Grundkörper
- 152: Durchmesser
- 154: Durchmesser
- 156: Breite
- 158: Länge
- 160: Länge
- 162: Ende
- 164: Ende
- 166: Aufnahme
- 168: Außenkontur
- 170: Innenkontur
- 172: Koppelstelle
- 174: Außengewinde
- 176: Kolben
- 178: Blutkuchen
- 180: Blutserum
- 182: axiale Richtung
- 184: Mittelachse

## Patentansprüche

1. Vorrichtung zur Herstellung zumindest einer biologisch aktiven Substanz mit zumindest einer Einheit (12), die einen Grundkörper (14) aufweist, **dadurch gekennzeichnet, dass** die Einheit (12) ein Container (16) ist.

2. Vorrichtung nach Anspruch 1, **dadurch gekennzeichnet, dass** der Container (16) flügelfrei ausgestaltet ist.

3. Vorrichtung nach Anspruch 1 oder 2, **dadurch gekennzeichnet, dass** der Container (16) kolbenlos ausgestaltet ist.

4. Vorrichtung nach einem der vorhergehenden Ansprüche, **gekennzeichnet durch** zumindest eine Verschlussvorrichtung (18), die irreversibel mit dem Grundkörper (14) verbunden ist.

5. Vorrichtung nach einem der vorhergehenden Ansprüche, **gekennzeichnet durch** zumindest eine Durchstechmembran (20).

6. Vorrichtung nach einem der vorhergehenden Ansprüche, **gekennzeichnet durch** zumindest ein Gasauslassventil (22) und/oder ein Gaseinlassventil (24).

7. Vorrichtung nach einem der vorhergehenden Ansprüche, **dadurch gekennzeichnet, dass** an dem Grundkörper (14) zumindest eine Verbindungseinheit (26) angeordnet ist.

8. Vorrichtung nach einem der vorhergehenden Ansprüche, **gekennzeichnet durch** zumindest einen Hohlraum (28), der zur sterilen Aufnahme von Blutbestandteilen (30) vorgesehen ist.

9. Vorrichtung nach einem der vorhergehenden Ansprüche, **gekennzeichnet durch** zumindest einen Scherkörper (32).

10. Vorrichtung nach Anspruch 8 und 9, **dadurch gekennzeichnet, dass** der zumindest eine Scherkörper (32) in dem zumindest einen Hohlraum (28) angeordnet ist.

11. Vorrichtung zumindest nach Anspruch 9, **dadurch gekennzeichnet, dass** der zumindest eine Scherkörper (32) von einer Perle (34) gebildet ist.

12. Vorrichtung zumindest nach Anspruch 8, **dadurch gekennzeichnet, dass** der zumindest eine Hohlraum (28) ein Fassungsvolumen größer als 50 ml aufweist.

13. System zur Herstellung zumindest einer biologisch aktiven Substanz mit zumindest einer Vorrichtung (10) nach den Ansprüchen 1 bis 12, **gekennzeichnet durch** zumindest ein weiteres Mittel (38) zur Blutentnahme und/oder Blutaufbereitung, das zusätzlich zu einem Container (16) als Bestandteil des Systems (36) vorgesehen ist.

14. Gesamtsystem mit zumindest einem ersten System (36) zur Herstellung zumindest einer biologisch aktiven Substanz, insbesondere nach Anspruch 13, und zumindest einem zweiten System (42) zur Herstellung zumindest einer biologisch aktiven Substanz, insbesondere nach Anspruch 13,
**dadurch gekennzeichnet, dass** sich das erste System (36) in zumindest einem Mittel (38) von dem zweiten System (42) unterscheidet.

15. Verfahren zum Nutzen einer Vorrichtung (10) nach den Ansprüchen 1 bis 12, **dadurch gekennzeichnet, dass**
a) einem tierischen Organismus mit einer Blutentnahmevorrichtung (44) Blutbestandteile (30) entnommen werden,
b) zumindest ein Teil dieser Blutbestandteile (30) zumindest einem Container (16) zugeführt wird und
c) zumindest ein Teil der Blutbestandteile (30) in einem Hohlraum (28) des Containers (16) zumindest einer Stresssituation zur Bildung zumindest einer biologisch aktiven Substanz ausgesetzt werden.
